# EUROPÄISCHE PATENTANMELDUNG

(11) **EP 1 491 228 A2**
(43) Veröffentlichungstag der Anmeldung: **29.12.2004**
(21) Anmeldenummer: 04014080.8
(22) Anmeldetag: 16.06.2004
(51) Int. Cl.: A61M 21/00

(54) **Vorrichtung zur Beschallung einer Person**

(30) Priorität: 23.06.2003 CH 10972003
(71) Anmelder: Sono dynamic AG, 8706 Meilen (CH)
(72) Erfinder: Suter Beat, 8706 Meilen (CH)
(74) Vertreter: Lauer, Joachim

(57) **Zusammenfassung**

Bei der Vorrichtung mit Liege zur insbesondere therapeutischen Beschallung einer auf der Liege liegenden Person weist der Schall Frequenzen im Infraschallbereich zwischen 2 und 20 Hz auf. Der Schall wird mit einem elektrodynamischen Lautsprecher erzeugt, welcher in einem Bereich der Liege angeordnet ist, in welchem in etwa der Schwerpunkt einer auf der Liege liegenden Person zu liegen kommt. Ziel der Infraschall-Beschallung ist es, Körperrhythmen im Frequenzbereich der Hirnströme zu stimulieren und dadurch körpereigene Prozesse zu synchronisieren. Speziell gedacht ist an die Förderung oder Minderung einer ggf. krankhaften Über- oder Unteraktivität besagter Hirnfrequenzen und an ein Training des Parasympathikus zur Regulation des Autonomen Systems (ZNS). Zugleich wirkt der Infraschall stoffwechselfördernd.

## Beschreibung

### TECHNISCHES GEBIET

Die vorliegende Erfindung betrifft eine Vorrichtung mit Liege zur Beschallung einer auf der Liege liegenden Person, wobei der Schall mit einem elektrodynamischen Lautsprecher erzeugt wird und Frequenzen im Bereich von 2 - 20 Hz umfasst, insbesondere zu therapeutischen Zwecken in den Bereichen Relaxation, Rehabilitation, Psycho- und Suchttherapie.

### STAND DER TECHNIK

Eine derartige Vorrichtung mit Liege und mit mehreren Lautsprechen ist bereits bekannt aus EP 0 422 253. Die Anordnung der Lautsprecher ist hier aber nicht optimal.

EP 0 244 508 beschreibt eine Vorrichtung mit Liege und nur einem Lautsprecher, welcher aber ebenfalls nicht optimal angeordnet ist. Ausserdem ist die Vorrichtung nur für höhere Frequenzen ab 30 Hz ausgelegt.

Weiter sind bekannt sogenannte sono dynamic®-Systeme der Anmelderin, welche einen aus Aluminiumrohren aufgebauten Würfeloktaeder umfassen, in welchem eine Liege mit horizontaler Liegefläche gehalten ist und in dessen Knotenpunkten oberhalb der Liege mehrere kleine Lautsprecher angeordnet sind. Über die Lautsprecher wird höhrbare Musik übertragen.

Daneben gibt es verschiedene Systeme welche mit allen möglichen Arten von Vibrationen arbeiten. Diesbezüglich lassen sich zwei Hauptgruppen unterscheiden:
- Gruppe A: Mechanisch über Motoren und Excenter erzeugte Vibrationen, welche auf flexible und feste Materialien übertragen werden;
- Gruppe B: Schallschwingungen, welche über Luft oder feste Verbindungen auf eine feste, flüssige oder flexible Materie übertragen werden.

Zur Gruppe A gehören vor allem die sogenannten Relaxsessel sowie Fussmassagegeräte.

Zur Gruppe B gehören Musikmassagesysteme der Somatron Corporation, 3405 Ellenwood Ln, Tampa, FL 33618 USA, die vorerwähnten Sono dynamic® Klangsysteme der Anmelderin, Musikbehandlungsysteme von Musica Medica, Illnauerstr. 10, CH-8307 Effretikon, sowie Music Vibration Dr. Donald E. Michel, P.O. Box 425 768 Texas Womans' University, Texas 76203 USA. Die Systeme dieser Gruppe arbeiten sämtlich mit Frequenzen im hörbaren Bereich, d.h. mit Frequenzen oberhalb ca. 20 Hz bis etwa 20'000 Hz.

### DARSTELLUNG DER ERFINDUNG

Viele Vorgänge im menschlichen Körper laufen repetitiv ab und unterliegen einem bestimmten Rhythmus. Die Frequenzen dieser Rhythmen, darunter bestimmte Hirnfrequenzen, sind eher klein und liegen im sogenannten Infraschallbereich, also bei Frequenzen, die mit dem menschlichen Ohr nicht mehr wahrgenommen werden können. Dennoch ist der Mensch in der Lage mit seiner Sensorik wie z.B. seinem Tastsinn, durchaus auch Schallschwingungen im Infraschallbereich wahrzunehmen und darauf zu reagieren.

Durch die Erfindung, wie sie in den Patentansprüchen gekennzeichnet ist, wird eine Vorrichtung mit Liege zur Beschallung einer auf der Liege liegenden Person geschaffen, bei welcher der Schall Frequenzen gerade im Infraschallbereich, also zwischen 2 und 20 Hz aufweist. Der Schall wird mit einem elektrodynamischen Lautsprecher erzeugt. Vorzugsweise beträgt die Frequenz der Schallwellen sogar nur bis etwa 10 Hz und insbesondere ca. 8 Hz. Der Lautsprecher ist in einem Bereich der Liege angeordnet, in welchem etwa der Schwerpunkt einer auf der Liege liegenden Person zu liegen kommt. Bei dieser Anordnung erfolgt die Wirkung der Infraschallwellenauf das Körperzentrum und ist so besonders wirksam.

Bei den vorgenannten Frequenzen soll es sich jeweils um vorherrschende Grundschwingung handeln, wobei gewisse Oberschwingungen durchaus vorhanden sein können. Die Oberschwingungen sollten intensitätsmässig auch in ihrer Summe gegenüber der Grundschwingung jedoch eher klein sein, was z.B. der Fall ist, wenn der erzeugte "Ton" insgesamt unhörbar ist.

Ziel der Infraschall-Beschallung ist es, Körperrhythmen der Person im Frequenzbereich der Hirnströme über Entrainment (Einkoppelung) zu stimulieren und dadurch körpereigene Prozesse zu synchronisieren. Speziell gedacht ist an die Förderung oder Minderung einer ggf. krankhaften Über- oder Unteraktivität besagter Hirnfrequenzen und an ein Training des Parasympathikus zur Regulation des Autonomen Systems (ZNS). Zugleich wirkt die Pulsation des Infraschalls über die Wassermoleküle in Zellen von Muskeln etc. z.B. stoffwechselfördernd.

Möglich erscheint ein medizinischer Einsatz zur Behandlung von
- chronischen undefinierten Muskelschmerzen,
- myofaszialen Rückenschmerzen,
- Spannungskopfschmerzen,
- Myogelosen,
- Schlafapnoe, autonomer Dysfunktion, und
- primärer und sekundärer Insomnie.

Im privaten Bereich kann die erfindungsgemässe Vorrichtung eingesetzt werden zur Vorsorge gegen:
- Autonome Balance,
- Stressbehandlung und Prophylaxe,
- Schlafprobleme, und
- Rückenschmerzen.
   Im sogenannten Wellnessbereich liegen Anwendungen zur
- tiefen Entspannung mit den unhörbaren Schwingungen, sowie zur
- Lockerung der Muskulatur als Vorbereitung bei Massagen.

Durch die Merkmale der Unteransprüche wird die erfindungsgemässe Vorrichtung in sinnvoller und wirkungsvoller Weise weitergebildet und ausgestaltet.

Die Liege der Vorrichtung umfasst danach eine im wesentlichen horizontale Liegefläche mit einer Matratzenauflage auf einer Unterkonstruktion, auf welcher die zu beschallende Person bequem und entspannt liegen kann. Der Lautsprecher ist knapp unterhalb der Matratze etwa im Bereich des Körperschwerpunkts der Person (Steissbein) angeordnet ist. Der Lautsprecher kann hierbei einfach an der Unterseite einer mit einer Schallöffnung versehenen Platte montiert sein, welchen einen Teil der Unterkonstruktion bildet. Es hat sich gezeigt, dass auf diese Weise zusammen mit einer geeignet dimensionierten Matratzenauflage der Infraschall auf den Körper wirksam übertragen werden.

Vorzugsweise wird mit niederschwelligen Schalldrücken gearbeitet. Es genügt, wenn der Verstärker, an welchen der Lautsprecher angeschlossen ist, ausgangsseitig eine Leistung bis etwa 20 Watt abgibt. Für spezielle Anwendungen kann es jedoch auch vorteilhaft sein, mit höheren Leistungen bis beispielsweise 100 Watt zu arbeiten. Weiter vorzugsweise sollte die Leistung variabel einstellbar sein.

### KURZE ERLÄUTERUNG DER FIGUREN

Die Erfindung soll nachfolgend anhand von Ausführungsbeispielen im Zusammenhang mit der Zeichnung näher erläutert werden. Es zeigen:
- Fig. 1: eine Vorrichtung nach der Erfindung im Schnitt mit einer darauf liegenden Person; und
- Fig. 2: in einer Detaildarstellung den Bereich um den Lautsprecher der Vorrichtung von Fig. 1.

### WEGE ZUR AUSFÜHRUNG DER ERFINDUNG

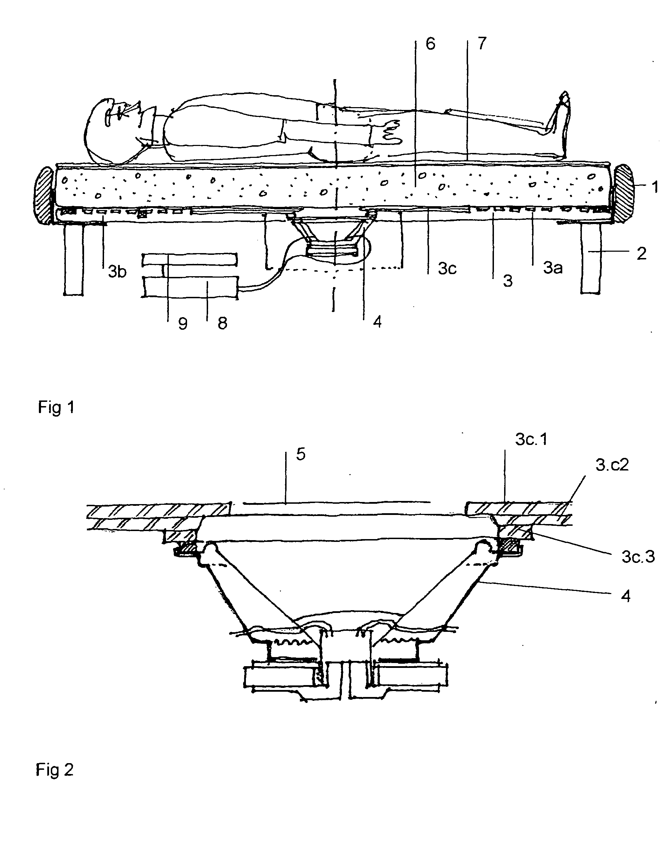

Die in Fig. 1 dargestellte Vorrichtung umfasst eine Liege mit einer Unterkonstruktion mit einem stabilen äusseren, umlaufenden Rahmen 1, welcher mit festen Füssen 2 versehen ist. Alternativ könnte auch ein höhenverstellbares Scherengestell oder dergleichen vorgesehen sein. In den Rahmen 1 eingelegt ist ein Rost 3. Dieser ist mit Latten im Bereich des Fussendes 3a und des Kopfendes 3b versehen und weist in seinem mittleren Bereich eine Platte 3c auf. An der Unterseite der Platte 3c ist ein Lautsprecher 4 mit Abstrahlrichtung nach oben montiert. Direkt oberhalb des Lautsprechers befindet sich in der Platte 3c ein Durchgangsloch 5 als Schallöffnung

Wie anhand der Detaildarstellung von Fig. 2 zu erkennen ist, ist die Platte 3c mehrlagig ausgebildet mit einer oberen Lage 3c.1, einer mittleren Lage 3c.2 sowie einem Ring 3c.2. Der Rand des Lautsprechers 4 ist mit dem Ring 3c.3 verbunden. Gegenüber dem Innendurchmesser des Rings 3c.3 ist die freie Öffnung der Lagen 3c.2 und 3.c 1 jeweils etwas kleiner gewählt, so dass sich die Schallöffnung nach oben hin verengt. Hierdurch wird der durch den Lautsprecher zunächst in einem sich nach oben erweiternden Konus abgestrahlte Schall wieder etwas nach innen umgelenkt, d.h. zur Achse hin etwas fokussiert. Der Durchmesser des Laufsprechers 5 kann z.B. 12 - 15 betragen.

Auf dem Rost 3 ist eine Matratzenauflage 6 vorhanden, bei welcher es sich insbesondere um eine solche aus geschlossenem, flexiblen Naturlatex-Material mit einer Dicke im Bereich zwischen 100 - 160 mm und einer Dichte im Bereich zwischen 0,06 und 0,76 kg/m³ handeln kann. Die Matratzenauflage ist in die Unterkonstruktion eingepasst und kann mit einer zusätzlichen Auflage 7 aus verschiedenen Wollqualitäten als Liegeunterlage versehen sein.

Die Position des Lautsprechers 4 ist quer in der Mittelachse und längs so gewählt, dass das sein Zentrum im wesentlichen mit dem Steissbein einer auf der Liege liegenden Person übereinstimmt.

Der Lautsprecher 4 ist an den Ausgang eines Verstärkers 8 angeschlossen, der wiederum mit einem Infraschallgenerator 9 gekoppelt ist.

Mit der Vorrichtung von Fig. 1 wird Infraschall mit eingegrenzter Frequenz im Bereich zwischen 2 - 20 Hz, vorzugsweise 2 - 10 Hz, insbesondere ca. 8 Hz, appliziert. Es wird bevorzugt mit niederschwelligen Schalldrücken von 0 bis 20 Watt (gemessen am Ausgang des Verstärkers 8) gearbeitet. Die Hauptübertragung erfolgt im Körperschwerpunkt (Steissbein) auf die liegende Person.

### BEZEICHNUNGSLISTE

- 1: Rahmen der Unterkonstruktion
- 2: Füsse am Rahmen
- 3: Rost
- 3a: Fussende
- 3b: Kopfende
- 3c: Platte im mittleren Bereich
- 4: Lautsprecher
- 5: Durchgangsloch, Schallöffnung
- 3c.1: obere Lage der Platte 3c
- 3c.2: mittlere Lage der Platte 3c
- 3c.3: Ring an der Platte 3c
- 6: Matratzenauflage
- 7: Auflage als Liegeunterlage
- 8: Verstärker
- 9: Infraschallgenerator

## Patentansprüche

1. Vorrichtung mit Liege zur Beschallung einer auf der Liege liegenden Person, wobei der Schall mit einem elektrodynamischen Lautsprecher erzeugt wird und Frequenzen im Bereich von 2 - 20 Hz umfasst, **dadurch gekennzeichnet, dass** der Lautsprecher in einem Bereich der Liege angeordnet ist, in welchem in etwa der Schwerpunkt einer auf der Liege liegenden Person zu liegen kommt.

2. Vorrichtung nach Anspruch 1, **dadurch gekennzeichnet, dass** der Schall Frequenzen im Bereich von 2 - 10 Hz, vorzugsweise von etwa 8 Hz, umfasst.

3. Vorrichtung nach Anspruch 1 oder 2, **dadurch gekennzeichnet, dass** die Liege eine im wesentlichen horizontale Liegefläche aufweist.

4. Vorrichtung nach einem der Ansprüche 1 - 3, **dadurch gekennzeichnet, dass** die Liege eine von einer Unterkonstruktion getragene Matratzenauflage aufweist.

5. Vorrichtung nach einem der Ansprüche 1 - 4, **dadurch gekennzeichnet, dass** der Lautsprecher unterhalb der Liege angeordnet ist.

6. Vorrichtung nach den Ansprüchen 4 und 5, **dadurch gekennzeichnet dass** der Lautsprecher an der Unterseite einer mit einer Schallöffnung versehenen Platte montiert ist, welche einen Teil der Unterkonstruktion bildet.

7. Vorrichtung nach Anspruch 6, **dadurch gekennzeichnet, dass** die Schallöffnung der Platte einen etwas geringeren Durchmesser aufweist, als die Membran des Lautsprechers.

8. Vorrichtung nach einem der Ansprüche 1 - 7, **dadurch gekennzeichnet, dass** der Lautsprecher an einen Verstärker angeschlossen ist, welcher ausgangsseitig eine Leistung im Bereich bis etwa 100 Watt , mindestens jedoch bis etwa 20 Watt, abgibt, welche Leistung vorzugsweise auch noch variabel einstellbar ist.
